# EUROPÄISCHE PATENTSCHRIFT

(11) **EP 0 944 573 B1**
(45) Veröffentlichungstag und Bekanntmachung des Hinweises auf die Patenterteilung: **12.09.2001**
(21) Anmeldenummer: 97951259.7
(22) Anmeldetag: 21.11.1997
(51) Int. Cl.: C07C 67/08, C07C 67/58, C07C 69/54

(54) **VERFAHREN ZUR HERSTELLUNG VON (METH)ACRYLSÄUREESTERN**
METHOD FOR THE PRODUCTION OF (METH)ACRYLIC ACID ESTERS
PROCEDE POUR LA PREPARATION D'ESTERS D'ACIDE (METH)ACRYLIQUE

(30) Priorität: 25.11.1996 DE 19648745
(43) Veröffentlichungstag der Anmeldung: 29.09.1999
(73) Patentinhaber: BASF AKTIENGESELLSCHAFT, 67056 Ludwigshafen (DE)
(72) Erfinder: AICHINGER, Heinrich, D-68199 Mannheim (DE); FRIED, Michael, D-69121 Heidelberg (DE); NESTLER, Gerhard, D-67061 Ludwigshafen (DE); DAMS, Albrecht, D-67157 Wachenheim (DE)
(74) Vertreter: Kinzebach, Werner, Dr.
(86) Internationale Anmeldenummer: EP9706514
(87) Internationale Veröffentlichungsnummer: WO9823577

(56) Entgegenhaltungen:
- EP-A- 0 463 434

## Beschreibung

Die Erfindung betrifft ein Verfahren zur Herstellung von Estern der Acrylsäure oder Methacrylsäure [(Meth)acrylsäure].

(Meth)acrylsäureester werden großtechnisch in der Regel durch Veresterung der (Meth)acrylsäure mit Alkanolen in Gegenwart von starken Säuren als Veresterungskatalysatoren (z.B. eine Mineralsäure, wie Schwefelsäure oder Phosphorsäure, Alkansulfonsäuren oder Arylsulfonsäuren) hergestellt. Solche Verfahren sind z.B. aus Kirk Othmer, "Encyclopedia of Chemical Technology", Vol. 1, S. 347-348 bekannt. Der Gehalt der Katalysatoren im Veresterungsgemisch kann sich in einer Größenordnung von Zehntelprozent bis zu mehreren Prozenten bewegen. Bei Verwendung von mehrbasischen Mineralsäuren als Katalysator kommt es leicht zur Veresterung der Mineralsäure mit dem anwesenden Alkanol unter Bildung des Monoesters, welcher der eigentliche Veresterungskatalysator ist. Das Reaktionsgemisch enthält nach Beendigung der Veresterung eine größere Menge dieses Monoesters.

Die als Katalysatoren verwendeten Säuren und deren gegebenenfalls gebildeten Ester müssen vor der Weiterverarbeitung aus dem Reaktionsgemisch beseitigt werden. In der Regel wird dies durch Auswaschen und Neutralisieren des Reaktionsgemisches mit Alkali- und Erdalkalilauge oder Carbonatlösungen erreicht. Dabei fallen Abwässer an, deren Beseitigung aufwendig und umweltbelastend ist. Wird Schwefelsäure als Katalysator verwendet, so bildet sich, wie erwähnt, vorwiegend der Monoester der Schwefelsäure mit dem betreffenden Alkanol. Die Salze der Schwefelsäuremonoester, insbesondere der Ester mit höheren Alkanolen, sind oberflächenaktiv und würden bei ihrer Entsorgung die Qualität der Abwässer aus dem Prozeß erheblich beeinträchtigen und einen nicht unbeträchtlichen Verlust an Wertprodukt verursachen. Aus wirtschaftlichen und ökologischen Gründen ist somit die Wiedergewinnung und Rückführung des Katalysators wünschenswert.

Der Stand der Technik umfaßt mehrere Verfahren, die jedoch alle mit erheblichen Nachteilen behaftet sind.

Die EP-A-0 609 127 beschreibt ein Verfahren zur Herstellung von (Meth)acrylsäureestern, wobei die Alkoholkomponente aus dem entsprechenden Schwefelsäuremonoester, der bei der Veresterung aus Schwefelsäure und dem Alkohol gebildet wird, durch saure Hydrolyse wiedergewonnen wird. Dieses Verfahren ist aufwendig, umweltbelastend und unwirtschaftlich.

Die CZ-B-179 808 beschreibt ein Verfahren zur Wiedergewinnung von Mineralsäuren aus Veresterungsgemischen durch Extraktion des Veresterungsgemisches mit Wasser, Konzentrierung der wäßrigen Phase durch Destillation und Rückführung der so erhaltenen konzentrierten wäßrigen Katalysatorlösung in die Veresterungsreaktion. Dieses Verfahren ist energieaufwendig.

Die EP-A-0 618 187 (≙US-A-5,386,052) beschreibt ein Verfahren zur Herstellung von (Meth)acrylsäureestern, wobei der Katalysator mit Wasser extrahiert und der Extrakt, gegebenenfalls nach destillativer Konzentrierung in die Veresterungsreaktion zurückgeführt wird. Es wird dabei jedoch besonders darauf hingewiesen, daß Schwefelsäure wegen der schlechten Extrahierbarkeit des Schwefelsäuremonoalkylesters als Katalysator ungeeignet ist, weil die große Wassermenge, die zur adäquaten Extraktion des Schwefelsäuremonoalkylesters nötig wäre, die Veresterungsreaktion negativ beeinträchtigen würde. Als Katalysator werden daher Alkyl- bzw. Arylsulfonsäuren verwendet (Spalte 2, Zeile 55ff), die jedoch wesentlich teurer sind als Schwefelsäure.

Der Erfindung liegt die Aufgabe zugrunde, ein technisch einfaches und wirtschaftliches Verfahren zur Herstellung von (Meth)acrylsäureestern zu entwickeln, das mit Schwefelsäure als Veresterungskatalysator auskommt und das es erlaubt, den Veresterungskatalysator (Schwefelsäure bzw. Monoalkylschwefelsäure) möglichst einfach und vollständig vom erhaltenen Reaktionsgemisch abzutrennen. Darüber hinaus soll der Katalysator wieder direkt, d.h. ohne zusätzliche destillative Konzentrierung, in die Veresterung rückführbar sein ohne die Veresterung zu beeinflussen.

Überraschenderweise wurde gefunden, daß der Katalysator aus dem Reaktionsgemisch (Veresterungsgemisch) extrahiert werden kann, wenn der Alkanolgehalt in dem Gemisch höchstens 5 Gew.-% beträgt.

Gegenstand der Erfindung ist daher ein Verfahren zur Herstellung von (Meth)acrylsäureestern durch Umsetzung von (Meth)acrylsäure mit C₄-C₁₂-Alkanolen, vorzugsweise C₄-C₁₀-Alkanolen, besonders bevorzugt C₄-C₈-Alkanolen in Gegenwart von Schwefelsäure oder eines Schwefelsäuremono-C₄-C₁₂-alkylesters als Katalysator, das dadurch gekennzeichnet ist, daß der Katalysator durch Extraktion mit Wasser aus dem Reaktionsgemisch regeneriert und die wäßrige Katalysatorlösung wieder in die Veresterung zurückgeführt wird, wobei die Konzentration an nicht umgesetztem Alkanol im zu extrahierenden Reaktionsgemisch höchstens 5 Gew.-%, bezogen auf das zu extrahierende Reaktionsgemisch, beträgt.

Es hat sich überraschenderweise gezeigt, daß der Alkanolgehalt einen großen Einfluß auf die Extrahierbarkeit des aus Schwefelsäure und Alkanol gebildeten Schwefelsäuremonoalkylesters, der als eigentlicher Veresterungskatalysator wirkt, hat (siehe Tabelle 1). Dadurch kann der Katalysator mit geringen Wassermengen extrahiert werden, so daß der Extrakt direkt wieder der Veresterung zugeführt werden kann. Vorzugsweise extrahiert man ein Reaktionsgemisch, dessen Alkanolgehalt ≤ 3 Gew.-% und insbesondere ≤ 1 Gew.-% ist.

Um einen Alkanolgehalt von höchstens 5 Gew.-% zu erreichen, wird vorzugsweise ein hoher Veresterungsumsatz herbeigeführt, z.B. durch Abdestillation des Reaktionswassers; und/oder ein geeignetes Verhältnis der Einsatzstoffe gewählt. Wenn der Restalkanolgehalt dann noch mehr als 5 Gew.-% beträgt, wird das Alkanol in einer herkömmlichen Destillationsapparatur (z.B. Kolonne mit Siebböden, Raschigringen, gerichteten Packungen etc.) abdestilliert. Überraschenderweise sind dabei trotz Anwesenheit des stark sauren Veresterungskatalysators keine säurekatalysierten Nebenreaktionen, wie Ether- oder Olefinbildung bzw. Addition des Alkanols an die Doppelbindung des (Meth)acrylats (Michael-Addition) in nennenswertem Umfang zu beobachten.

Die Destillation wird in üblicher Weise durchgeführt; die Destillationsbedingungen richten sich nach der Art des eingesetzten Alkanols.

Das Abdestillieren des Alkanols erfolgt vorzugsweise bis zu einem Gehalt an Restalkanol im Reaktionsgemisch, der die Extraktion des Katalysators (Schwefelsäure) mit Wasser problemlos ermöglicht. Insbesondere beträgt der Restalkanolgehalt ≤ 5 Gew.-%, vorzugsweise ≤ 3 Gew.-%, besonders bevorzugt ≤ 1 Gew.-%, bezogen auf das Reaktionsgemisch.

Vorzugsweise werden die Bedingungen für die Extraktion des Katalysators aus dem Veresterungsgemisch so gewählt, daß die Katalysatorkonzentration (Schwefelsäure und Schwefelsäuremonoalkylester) in der wäßrigen Phase mindestens 20 Gew.-%, insbesondere mindestens 25 Gew.-%, bezogen auf den wäßrigen Extrakt, und der Extraktionsgrad mindestens 70 Gew.-%, insbesondere mindestens 80 Gew.-%, bezogen auf die Katalysatormenge im Reaktionsgemisch, beträgt. Um dies zu erreichen, verwendet man zur Extraktion etwa 5 bis 20 Gew.-% Wasser, insbesondere etwa 8 bis 15 Gew.-% Wasser, bezogen auf das Gesamtgewicht des Veresterungsgemisches. Der erhaltene Extrakt kann wieder der Veresterung zugeführt werden, ohne ihn aufzukonzentrieren.

Die Durchführung der Extraktion kann in an sich bekannter Weise erfolgen. Vorzugsweise extrahiert man im Gegenstrom, z.B. in Kolonnen ohne Energieeintrag, gepulsten Kolonnen, Kolonnen mit Einsätzen, Mixer-Settler-Apparaturen oder in statischen Mischern.

Die Extraktion kann bei Umgebungstemperatur oder höherer Temperatur durchgeführt werden, zweckmäßigerweise jedoch bei einer Temperatur im Bereich von etwa 15 bis 40°C.

Die Veresterung wird im wesentlichen in üblicher Weise durchgeführt, d.h. durch Umsetzung von (Meth)acrylsäure mit einem C₄-C₁₂-Alkanol in Gegenwart eines Katalysators und bei erhöhter Temperatur. Das molare Verhältnis Alkanol:Acrylsäure bzw. Methacrylsäure beträgt im allgemeinen 1:0,8-1,2. C₄-C₁₂-Alkanole sind beispielsweise Pentanol, Hexanol, Heptanol, Octanol, 2-Ethylhexanol, Nonanol, 2-Propylheptanol, Decanol, Undecanol, Dodecanol, sowie vorzugsweise Butanole, insbesondere n-Butanol. Die Schwefelsäurekonzentration im Reaktionsgemisch beträgt in der Regel 0,5 bis 10 Gew.-%, vorzugsweise 1 bis 5 Gew.-%, bezogen auf den Gesamtansatz.

Als Polymerisationsinhibitoren werden z.B. Phenothiazin, Hydrochinon, Hydrochinonmonomethylether bzw. Gemische davon und gegebenenfalls Luft (0,1 bis 10 l/h x l) in einer Menge von 100 bis 5000 ppm, bezogen auf das Reaktionsgemisch, verwendet.
Als Schleppmittel zur Entfernung von Wasser aus dem Reaktionsgemisch können im erfindungsgemäßen Verfahren gesättigte Kohlenwasserstoffe (z.B. Cyclohexan) oder Aromaten (z.B. Toluol) verwendet werden; vorzugsweise wird die Reaktion aber ohne zusätzliches Schleppmittel durchgeführt.

Die Reaktionstemperatur liegt im allgemeinen bei etwa 70 bis 160°C, vorzugsweise bei 90 bis 130°C.

Die Reaktionszeit beträgt im allgemeinen etwa 1 bis 10, vorzugsweise 1 bis 6 Stunden.

Die Reaktion kann unter Normal-, Unter- oder Überdruck durchgeführt werden. Vorzugsweise wir der Druck so eingestellt, daß während der Veresterung das gebildete Wasser abdestilliert, z.B. in Form eines Gemisches aus Wasser, C₄-C₁₂-Alkanol und Ester (die organischen Komponenten werden dabei wieder der Veresterung zugeführt. Die Veresterung kann kontinuierlich oder diskontinuierlich durchgeführt werden, wobei die kontinuierliche Reaktionsführung bevorzugt ist.

Die Veresterung wird in üblichen Apparaturen durchgeführt, z.B. in einer Veresterungseinheit aus einem oder mehreren beheizbaren Rührreaktoren (Kaskade), die gegebenenfalls mit Kolonnen, Kondensatoren und Phasentrenngefäßen ausgerüstet sind. Der Reaktorinhalt wird durch Rühren oder andere übliche und geeignete Maßnahmen durchmischt.

Falls nach der Extraktion des Katalysators noch eine Extraktion/ Neutralisation der Restsäuren (Katalysator und (Meth)acrylsäure) mit Hilfe einer wäßrigen Base notwendig ist, kann diese in einer herkömmlichen Extraktionsapparatur (siehe oben) erfolgen, wobei der Basenbedarf aufgrund des hohen Extraktionsgrades des Katalysators gering ist und die Extraktion überraschenderweise ohne die in der EP-A-0 566 074 beschriebenen Phasentrennprobleme verläuft.

Die Isolierung des Esters aus dem von Katalysator und gegebenenfalls Restcarbonsäure und Leichtsieder befreiten Reaktionsgemisch erfolgt in üblicher Weise, insbesondere destillativ, z.B. durch Destillation in einer Siebbodenkolonne.

Die Erfindung wird durch das nun folgende Beispiel erläutert, ohne sie einzuschränken. Bei den Prozentangaben handelt es sich um Gewichtsprozente.

Eine Rührkesselkaskade bestehend aus 3 Rührreaktoren mit je 1 l Reaktionsvolumen, die mit Kolonne, Kondensator und Phasentrenngefäß ausgerüstet sind, wurde mit 558 g Acrylsäure, 648 g n-Butanol, 16 g Schwefelsäure und 1 g Phenothiazin pro Stunde beschickt. Die Reaktionstemperatur in den Reaktoren betrug 106°C, 118°C bzw. 123°C, der Druck 700 mbar. Am Kopf der Kolonne fiel ein Gemisch aus Wasser, n-Butanol und n-Butylacrylat an, das in eine wäßrige und eine organische Phase zerfiel, wobei die organische Phase der Kolonne als Rücklauf zugeführt wurde.

Der Reaktionsaustrag (1070 g/h) enthielt gemäß Analyse:
90,2% n-Butylacrylat
2,7% n-Butanol
0,5% Acrylsäure
2,2% Butylschwefelsäure
Rest: Nebenprodukte, Polymere, Oligomere, Phenothiazin
Acrylsäureumsatz: 99%, Umwandlung 98%

Der auf 25°C abgekühlte Reaktionsaustrag wurde in einer Mixer-Settler-Apparatur mit 90 g/h Wasser bei ca. 25°C extrahiert. Die wäßrige Phase (97 g/h) enthielt 20,5% Schwefelsäuremono-n-butylester (85% Rückgewinnung) und 0,5% Schwefelsäure.

Diese wäßrige Phase wurde in den unteren Teil der Destillationskolonne des ersten Reaktors zurückgefahren, wobei die Zugabe von frischer Schwefelsäure auf 1,3 g/h reduziert wurde. Der Acrylsäureumsatz betrug weiterhin 99%, die Umwandlung 98%.

Die Abhängigkeit des Katalysator-Extraktionsgrades vom n-Butanolgehalt des der Extraktion zugeführten Reaktionsgemisches wurde ermittelt, indem durch Veresterung von Acrylsäure mit n-Butanol in Gegenwart von Schwefelsäure hergestellte Veresterungsgemische, die unterschiedliche n-Butanolgehalte aufwiesen, in einem Scheidetrichter einmal mit 10 Gew.-% Wasser bei 25°C extrahiert wurden. Die Ergebnisse dieser Versuche sind in Tabelle 1 dargestellt und belegen die Bedeutung einer Reaktionsführung, die den Gehalt an nicht umgesetztem Alkanol unter 5%, vorzugsweise bei 0,1% bis 3%, hält.

**Tabelle 1**

| | | | | |
|---|---|---|---|---|
| n-Butanolgehalt | 0,1% | 2,5% | 5,0% | 10% |
| Kat.-gehalt | 1,93% | 1,88% | 1,83% | 1,75% |
| Extraktionsgrad | 89% | 88% | 71% | 55% |
| Restkat.-gehalt | 0,22% | 0,23% | 0,53% | 0,97% |

## Patentansprüche

1. Verfahren zur Herstellung von (Meth)acrylsäureestern durch Umsetzung von (Meth)acrylsäure mit einem C₄-C₁₂-Alkanol in Gegenwart von Schwefelsäure oder eines Schwefelsäuremono-C₄-C₁₂-alkylesters als Katalysator, **dadurch gekennzeichnet, daß** der Katalysator durch Extraktion mit Wasser aus dem Reaktionsgemisch regeneriert wird, wobei die Konzentration an nicht umgesetztem Alkanol im zu extrahierenden Reaktionsgemisch höchstens 5 Gew.-%, bezogen auf das zu extrahierende Reaktionsgemisch, beträgt.

2. Verfahren nach Anspruch 1, **dadurch gekennzeichnet, daß** die Alkanolkonzentration 0,1 bis 3 Gew.-% beträgt.

3. Verfahren nach Anspruch 1 oder 2, **dadurch gekennzeichnet, daß** die wäßrige Lösung des durch Extraktion mit Wasser aus dem Reaktionsgemisch regenerierten Katalysators wieder in die Veresterung zurückgeführt wird.

4. Verfahren nach einem der Ansprüche 1 bis 3, **dadurch gekennzeichnet, daß** die Extraktion des Katalysators so durchgeführt wird, daß der Extraktionsgrad mindestens 70 Gew.-%, insbesondere mindestens 80 Gew.-%, bezogen auf die Katalysatormenge im Reaktionsgemisch, beträgt.

5. Verfahren nach einem der Ansprüche 1 bis 4, **dadurch gekennzeichnet, daß** die Extraktion des Katalysators so durchgeführt wird, daß die Katalysatorkonzentration in der Wasserphase mindestens 20 Gew.-%, insbesondere mindestens 25 Gew.-%, bezogen auf den wäßrigen Extrakt, beträgt.

6. Verfahren nach einem der Ansprüche 1 bis 5, **dadurch gekennzeichnet, daß** die Extraktion bei 15 bis 40°C durchgeführt wird.

7. Verfahren nach einem der Ansprüche 1 bis 6, **dadurch gekennzeichnet, daß** die Extraktion im Gegenstrom oder in einem statischen Mischer durchgeführt wird.

8. Verfahren nach einem der Ansprüche 1 bis 7, **dadurch gekennzeichnet, daß** als Alkanol n-Butanol oder Isobutanol verwendet wird.

9. Verfahren nach einem der Ansprüche 1 bis 8, **dadurch gekennzeichnet, daß** die Reaktionstemperatur 70 bis 160°C, insbesondere 90 bis 130°C beträgt.

10. Verfahren nach einem der Ansprüche 1 bis 9, **dadurch gekennzeichnet, daß** die Reaktionszeit 1 bis 10, insbesondere 1 bis 6 Stunden beträgt.

## Claims

1. A process for preparing (meth)acrylic esters by reacting (meth)acrylic acid with a C₄-C₁₂-alkanol in the presence of sulfuric acid or a mono-C₄-C₁₂-alkyl sulfate as catalyst, which comprises regenerating the catalyst by extraction with water from the reaction mixture, the concentration of unreacted alkanol in the reaction mixture to be extracted being not more than 5% by weight, based on the reaction mixture to be extracted.

2. A process as claimed in claim 1, wherein the alkanol concentration is from 0.1 to 3% by weight.

3. A process as claimed in claim 1 or 2, wherein the aqueous solution of the catalyst regenerated by extraction with water from the reaction mixture is recycled back to the esterification.

4. A process as claimed in one of claims 1 to 3, wherein the catalyst is extracted in such a manner that the degree of extraction is at least 70% by weight, in particular at least 80% by weight, based on the amount of catalyst in the reaction mixture.

5. A process as claimed in one of claims 1 to 4, wherein the catalyst is extracted in such a manner that the catalyst concentration in the water phase is at least 20% by weight, in particular at least 25% by weight, based on the aqueous extract.

6. A process as claimed in one of claims 1 to 5, wherein the extraction is performed at from 15 to 40°C.

7. A process as claimed in one of claims 1 to 6, wherein the extraction is carried out in counter-current or in a static mixer.

8. A process as claimed in one of claims 1 to 7, wherein the alkanol used is n-butanol or isobutanol.

9. A process as claimed in one of claims 1 to 8, wherein the reaction is performed at from 70 to 160°C, in particular from 90 to 130°C.

10. A process as claimed in one of claims 1 to 9, wherein the reaction time is from 1 to 10, in particular from 1 to 6, hours.

## Revendications

1. Procédé de préparation d'esters de l'acide (méth)acrylique par réaction d'acide (méth)acrylique avec un alcanol en C₄-C₁₂ en présence d'acide sulfurique ou d'un sulfate de monoalkyle en C₄-C₁₂ servant de catalyseur, **caractérisé en ce que** le catalyseur est régénéré par extraction avec de l'eau à partir du mélange réactionnel, la concentration de l'alcanol n'ayant pas réagi dans le mélange réactionnel devant être extrait étant d'au plus 5 % en poids par rapport au mélange réactionnel à extraire.

2. Procédé selon la revendication 1, **caractérisé en ce que** la concentration de l'alcanol est de 0,1 à 3 % en poids.

3. Procédé selon la revendication 1 ou 2, **caractérisé en ce que** la solution aqueuse du catalyseur régénérée par extraction à partir du mélange réactionnel à l'aide d'eau est renvoyée à l'estérification.

4. Procédé selon l'une des revendications 1 à 3, **caractérisé en ce que** l'extraction du catalyseur est mise en oeuvre de telle sorte que le degré d'extraction soit d'au moins 70 % en poids, en particulier d'au moins 80 % en poids par rapport à la quantité de catalyseur se trouvant dans le mélange réactionnel.

5. Procédé selon l'une des revendications 1 à 4, **caractérisé en ce que** l'extraction du catalyseur est mise en oeuvre de façon que la concentration du catalyseur dans la phase aqueuse soit d'au moins 20 % en poids, en particulier d'au moins 25 % en poids par rapport à l'extrait aqueux.

6. Procédé selon l'une des revendications 1 à 5, **caractérisé en ce que** l'extraction est mise en oeuvre à une température de 15 à 40°C.

7. Procédé selon l'une des revendications 1 à 6, **caractérisé en ce que** l'extraction est mise en oeuvre à contre-courant ou dans un mélangeur statique.

8. Procédé selon l'une des revendications 1 à 7, **caractérisé en ce qu'**on utilise en tant qu'alcanol du n-butanol ou de l'isobutanol.

9. Procédé selon l'une des revendications 1 à 8, **caractérisé en ce que** la température de la réaction est de 70 à 160°C, en particulier de 90 à 130°C.

10. Procédé selon l'une des revendications 1 à 9, **caractérisé en ce que** la durée de la réaction est de 1 à 10 et en particulier de 1 à 6 heures.
